# EUROPEAN PATENT SPECIFICATION

(11) **EP 1 542 166 B1**
(45) Date of publication and mention of the grant of the patent: **08.08.2007**
(21) Application number: 04257583.7
(22) Date of filing: 06.12.2004
(51) Int. Cl.: G06T 11/00

(54) **Three-dimension back-projection method and apparatus, and x-ray CT apparatus**
Verfahren und Vorrichtung zur dreidimensionalen Rückprojektion und Röntgenstrahlencomputertomograph
Procédé et dispositif pour la rétroprojection tri-dimensionelle et appareil de tomographie par ordinateur

(30) Priority: 10.12.2003 JP 2003411283
(43) Date of publication of application: 15.06.2005
(73) Proprietor: GE Medical Systems Global Technology Company LLC, Waukesha, Wisconsin 53188-1696 (US)
(72) Inventor: Nishide, Akihiko, Hino-shi Tokyo 191-8503 (JP); Hagiwara, Akira, Hino-shi Tokyo 191-8503 (JP)
(74) Representative: Pedder, James Cuthbert

(56) References cited:
- EP-A- 1 445 735
- HEIN I A ET AL: "Double-centering method for increasing efficiency of cone-beam X-ray CT reconstruction" 2001 IEEE NUCLEAR SCIENCE SYMPOSIUM CONFERENCE RECORD. / 2001 IEEE NUCLEAR SCIENCE SYMPOSIUM AND MEDICAL IMAGING CONFERENCE. SAN DIEGO, CA, NOV. 4 - 10, 2001, IEEE NUCLEAR SCIENCE SYMPOSIUM CONFERENCE RECORD, NEW YORK, NY : IEEE, US, vol. 3, 4 November 2001 (2001-11-04), pages 1727-1731, XP002278355 ISBN: 0-7803-7324-3

## Description

The present invention relates to a three-dimensional back-projection method and apparatus, and an X-ray CT (computed tomography) apparatus, and to image reconstruction according to a three-dimensional back-projection method in a conventional scan (axial scan).

In conducting image reconstruction for a conventional scan (axial scan) based on a three-dimensional back-projection method, processing is conducted for each row in a multi-row detector by an image reconstruction method common to the rows (for example, see Patent Document 1).

[Patent Document 1] Japanese Patent Application Laid Open No. 2003-225230 (pages 9-10, Figures 1-2)

HEIN I A ET AL: "Double-centering method for increasing efficiency of cone-beam X-ray CT reconstruction" 2001 IEEE NUCLEAR SCIENCE SYMPOSIUM CONFERENCE RECORD. / 2001 IEEE NUCLEAR SCIENCE SYMPOSIUM AND MEDICAL IMAGING CONFERENCE. SAN DIEGO, CA, NOV. 4 - 10, 2001 IEEE NUCLEAR SCIENCE SYMPOSIUM CONFERENCE RECORD, NEW YORK, NY: IEEE, US, vol 3, 4 November 2001 (2001-11-04), pages 1727-1731, XP002278355 ISBN: 0-7803-7324-3 describes a method of increasing the efficiency of hardware based circular and helical cone beam X-ray CT reconstruction.

The image reconstruction based on the three-dimensional back-projection method generally poses a problem in that it requires a larger amount of calculation than that of image reconstruction based on two-dimensional back-projection, and is more time-consuming.

Therefore, the present invention seeks to provide a three-dimensional back-projection method and apparatus that requires a smaller amount of calculation and is less time-consuming, and an X-ray CT apparatus comprising such a three-dimensional back-projection apparatus.

Various aspects and embodiments of the present invention are defined in the appended claims.

Preferably, the back-projected pixel data D2 is determined as the result of weighted addition on back-projected pixel data D2 at a certain rotation angle (view) and back-projected pixel data D2 at an opposite rotation angle (view) with weighting factors wₐ and *w_{b} (wₐ* + *w_{b}* = 1) that depend upon the angle formed by a straight line connecting a pixel on the reconstruction field at these views and an X-ray focal spot with respect to the plane of the reconstruction field, so that the back-projected pixel data D2 can be appropriately determined.

The invention will now be described in greater detail, by way of example, with reference to the drawings, in which:-
Figure 1 is a block diagram showing an X-ray CT apparatus in accordance with one embodiment of the present invention.
Figure 2 is an explanatory diagram showing a rotation of an X-ray tube and a multi-row detector.
Figure 3 is an explanatory diagram showing a cone beam.
Figure 4 is a flow chart showing a general operation of the X-ray CT apparatus in accordance with one embodiment of the present invention.
Figure 5 is a flow chart showing details of three-dimensional image reconstruction processing.
Figure 6 is a conceptual diagram showing lines on a reconstruction field projected in the direction of X-ray transmission.
Figure 7 is a conceptual diagram showing lines projected onto a detector plane.
Figure 8 is a conceptual diagram showing projection data Dr on lines at a rotation angle = 0° projected onto a projection plane.
Figure 9 is a conceptual diagram showing projection line data Dp on the lines at the rotation angle = 0° projected onto the projection plane.
Figure 10 is a conceptual diagram showing image-positional line data Df on the lines at the rotation angle = 0° projected onto the projection plane.
Figure 11 is a conceptual diagram showing high density image-positional line data Dh on the lines at the rotation angle = 0° projected onto the projection plane.
Figure 12 is a conceptual diagram showing back-projected pixel data D2 on lines on a reconstruction field at the rotation angle = 0°.
Figure 13 is a conceptual diagram showing back-projected pixel data D2 of pixels on the reconstruction field at the rotation angle = 0°.
Figure 14 is a conceptual diagram showing projection data Dr on lines at a rotation angle = 90° projected onto a projection plane.
Figure 15 is a conceptual diagram showing projection line data Dp on the lines at the rotation angle = 90° projected onto the projection plane.
Figure 16 is a conceptual diagram showing image-positional line data Df on the lines at the rotation angle = 90° projected onto the projection plane.
Figure 17 is a conceptual diagram showing high density image-positional line data Dh on the lines at the rotation angle = 90° projected onto the projection plane.
Figure 18 is a conceptual diagram showing backprojected pixel data D2 on lines on a reconstruction field at the rotation angle = 90°.
Figure 19 is a conceptual diagram showing backprojected pixel data D2 of pixels on the reconstruction field at the rotation angle = 90°.
Figure 20 is an explanatory diagram showing backprojected data D3 obtained by adding the backprojected pixel data D2 on a pixel-by-pixel basis for all views.
Figure 21 is an explanatory diagram showing that sufficient image quality can be attained for a small cone angle even if the number of planar projection lines is small.

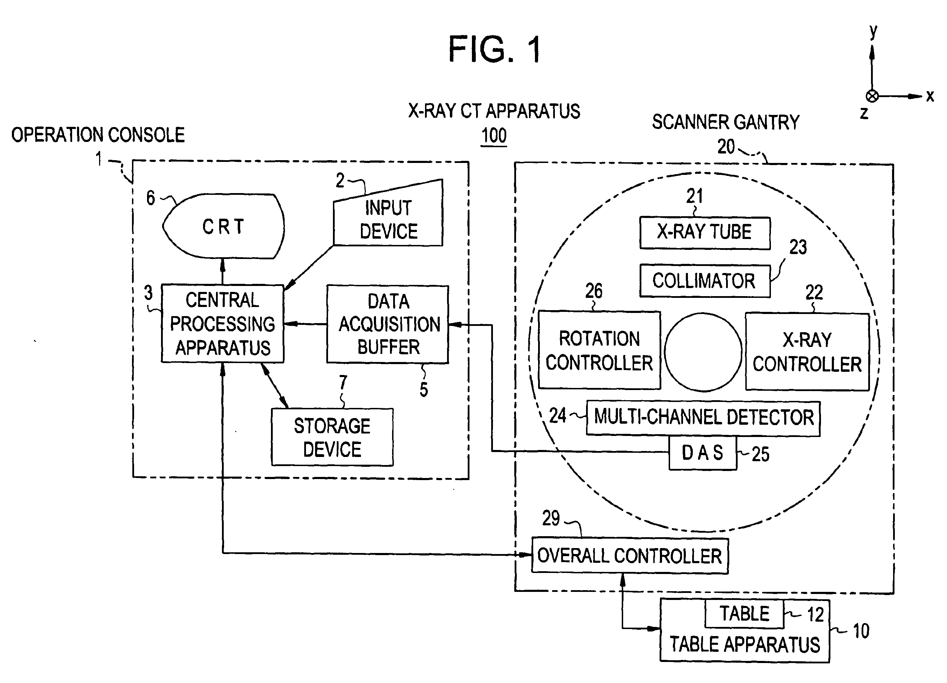

The best mode for carrying out the present invention will now be described in detail with reference to the accompanying drawings. It should be noted that the present invention is not limited to the best mode for carrying out the present invention. Figure 1 shows a block diagram of an X-ray CT apparatus. The present apparatus is an example of the best mode for carrying out the present invention. The configuration of the present apparatus represents an example of the best mode for carrying out the present invention with respect to the three-dimensional back-projection apparatus or X-ray CT apparatus of the present invention. The operation of the present apparatus represents an example of the best mode for carrying out the present invention with respect to the three-dimensional back-projection method of the present invention.

The X-ray CT apparatus 100 comprises an operation console 1, an imaging table 10, and a scan gantry 20. The operation console 1 comprises an input device 2 for accepting inputs by a human operator, a central processing apparatus 3 for executing three-dimensional back-projection processing in accordance with the present invention etc., a data collection buffer 5 for collecting projection data acquired at the scan gantry 20, a CRT 6 for displaying a CT image reconstructed from the projection data, and a storage device 7 for storing programs, data, and X-ray CT images.

The table apparatus 10 comprises a cradle 12 for laying thereon a subject and transporting the subject into/out of a bore (cavity portion) of the scan gantry 20. The cradle 12 is driven by a motor built in the table apparatus 10.

The scan gantry 20 comprises an X-ray tube 21, an X-ray controller 22, a collimator 23, a multi-row detector 24, a DAS (data acquisition system) 25, a rotation controller 26 for rotating the X-ray tube 21 and the like around the body axis of the subject, and a control interface 29 for communicating control signals etc. with the operation console 1 and imaging table 10. Also via the control interface 29, the X-ray controller 22, collimator 23 and rotation controller 26 are controlled by the central processing apparatus 3.

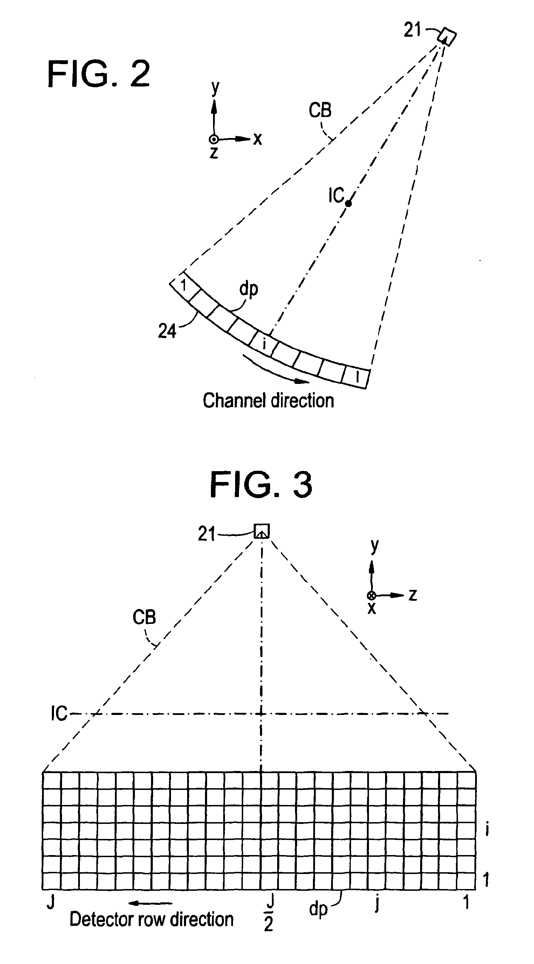

The following description will be made on a conventional scan (axial scan). Figures 2 and 3 are explanatory diagrams of the X-ray tube 21 and multi-row detector 24. The X-ray tube 21 and multi-row detector 24 rotate around a center of rotation IC. Representing the vertical direction as y-direction, the horizontal direction as x-direction, and a direction perpendicular to these directions as z-direction, the plane of rotation of the X-ray tube 21 and multi-row detector 24 is the x-y plane. The direction of translation of the cradle 12 is the z-direction. In place of the multi-row detector 24, a planar X-ray detector may be employed.

The X-ray tube 21 generates an X-ray beam generally referred to as cone beam CB. The direction of the center axis of the cone beam CB parallel to the y-direction is defined as a rotation angle = 0°. The multi-row detector 24 has 256 detector rows, for example. Each detector row has 1,024 channels, for example.

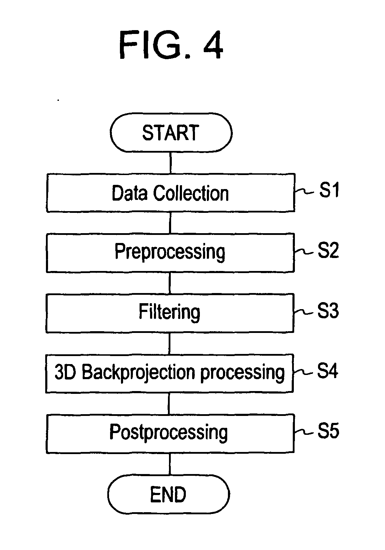

Figure 4 is a flow chart showing the general operation of the X-ray CT apparatus 100. At Step S1, projection data D0(z, *view, j, i)* represented by the table's rectilinear motion position z, view angle *view,* detector row index *j* and channel index *i* is collected while rotating the X-ray tube 21 and multi-row detector 24 around the subject to be imaged. The data collection is conducted by the scan gantry 20. The scan gantry 20 is an example of the scanning means of the present invention

At Step S2, the projection data D0(z, *view, j, i)* is subjected to pre-processing (offset correction, log correction, X-ray dose correction and sensitivity correction). At Step S3, the pre-processed projection data D0(z, *view, j, i*) is filtered. Specifically, the data is subjected to Fourier transformation, multiplied by a filter (reconstruction function), and then subjected to inverse Fourier transformation.

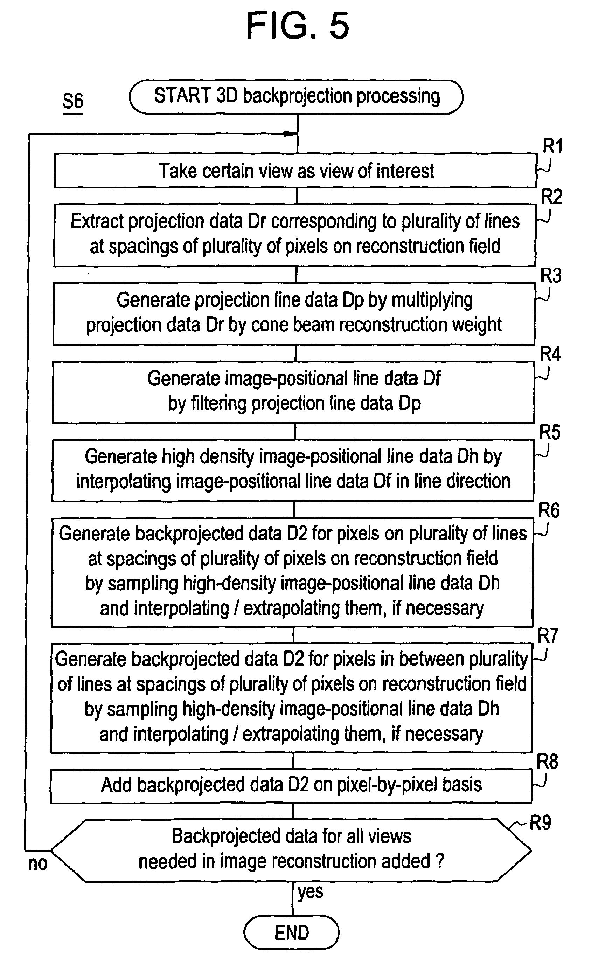

At Step S4, the filtered projection data D0(z. *view, j, i)* is subjected to three-dimensional back-projection processing to determine back-projected data D3(x, y). The three-dimensional back-projection processing will be discussed below with reference to Figure 5. At Step S5, the back-projected data D3(x, y) is subjected to post-processing to obtain a CT image.

Figure 5 is a flow chart showing details of the three-dimensional back-projection processing (Step S4 in Figure 4). The flow chart represents an operation of the central processing apparatus 3. At Step R1, one view is taken as a view of interest from among all views needed in reconstruction of a CT image (i.e., views for 360° or for "180° + fan angle"). At Step R2, projection data Dr corresponding to a plurality of parallel lines at spacings of a plurality of pixels on a reconstruction field are extracted from among the projection data D0(z, *view, j, i)* at the view of interest.

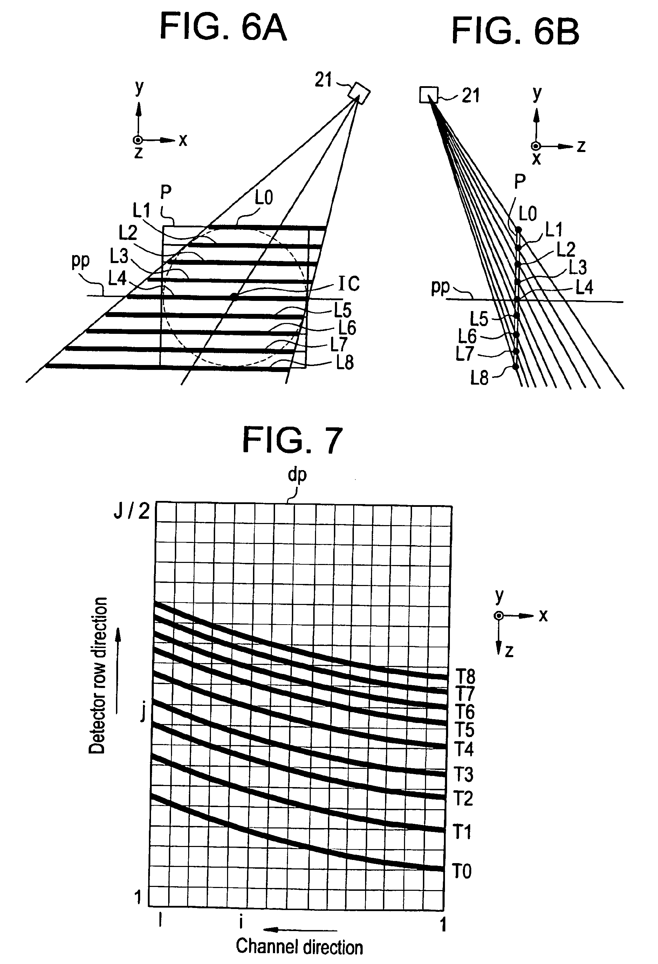

Figure 6 exemplarily shows a plurality of parallel lines L0 - L8 on the reconstruction field P. The number of lines is 1/64 - 1/2 of the maximum number of pixels in the reconstruction field in a direction orthogonal to the lines. For example, if the number of pixels in the reconstruction field P is 512x512. the number of lines is nine.

Moreover, the line direction is defined as the x-direction for -45° ≤ rotation angle < 45° (or a view angle range mainly including the range and also including its vicinity) and 135° ≤ rotation angle < 225° (or a view angle range mainly including the range and also including its vicinity). The line direction is defined as the y-direction for 45° ≤ rotation angle < 135° (or a view angle range mainly including the range and also including its vicinity) and 225° ≤ rotation angle < 315° (or a view angle range mainly including the range and also including its vicinity). Furthermore, a projection plane pp is assumed to pass through the center of rotation IC and be parallel to the lines L0 - L8.

Figure 7 shows lines T0 - T8 formed by projecting the plurality of parallel lines L0 - L8 onto a detector plane dp in the direction of X-ray transmission. The direction of X-ray transmission is determined depending upon the geometrical position of the X-ray tube 21, multi-row detector 24 and lines L0 - L8 (including the distance in the z-axis direction from the x-y plane passing through the center in the z-axis direction of the multi-row detector 24 to the image reconstruction field P, and the positions of the lines L0 - L8 formed by a set of pixel points on the image reconstruction plane P); since the position z of the projection data D0(z, *view, j, i*) in the direction of the table's rectilinear motion is known, the direction of X-ray transmission can be accurately determined.

The projection data Dr corresponding to the lines L0 - L8 can be obtained by extracting projection data at the detector row *j* and channel *i* corresponding to the lines T0 - T8 projected onto the detector plane dp. The projection data Dr is obtained by interpolation or extrapolation, if necessary.

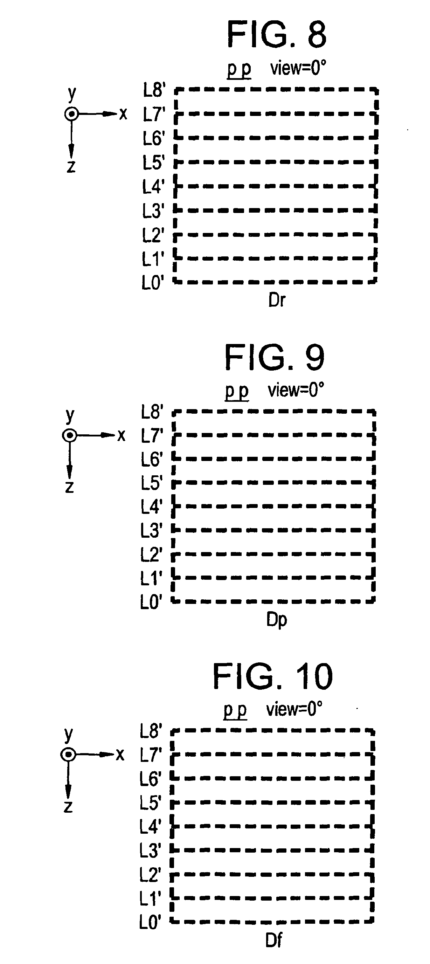

Now lines L0' - L8' formed by projecting the lines T0 - T8 onto the projection plane pp in the direction of X-ray transmission are assumed as shown in Figure 8, and the projection data Dr are arranged over the lines L0' - L8' based on the z-axis coordinate information.

Referring again to Figure 5, at Step R3, the projection data Dr of the lines L0' - L8' are multiplied by a cone beam reconstruction weight to generate projection line data Dp as shown in Figure 9. The cone beam reconstruction weight is (*r*1/*r*0)², where r0 is the distance from the focal spot of the X-ray tube 21 to the j-th detector row and the i-th channel of the multi-row detector 24 corresponding to projection data Dr, and r1 is the distance from the focal spot of the X-ray tube 21 to a point on the reconstruction field corresponding to the projection data Dr.

At Step R4, the projection line data Dp are filtered. Specifically, the projection line data Dp are subjected to FFT, multiplied by a filter function (reconstruction function), and subjected to inverse FFT to generate image-positional line data Df as shown in Figure 10.

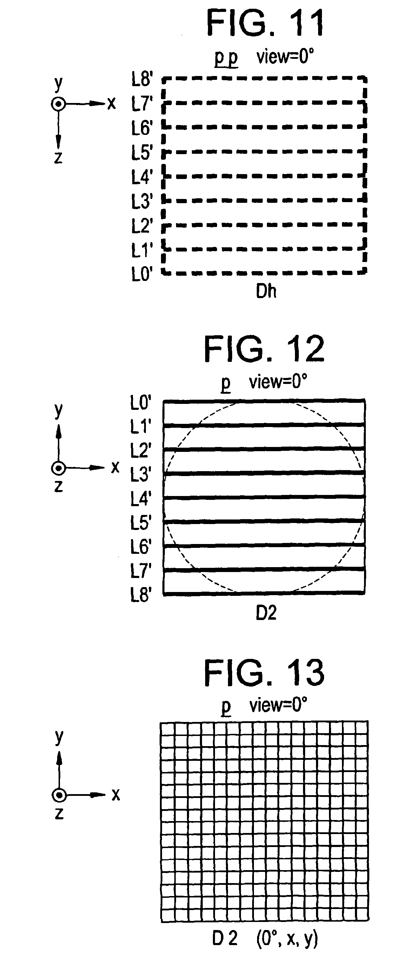

At Step R5, the image-positional line data Df is interpolated in the line direction to generate high-density image-positional line data Dh as shown in Figure 11. The data density of the high-density image-positional line data Dh is 8 - 32 times the maximum number of pixels in the reconstruction field in the line direction. For example, if the factor is 16 and the number of pixels in the reconstruction field P is 512x512, the data density is 8,192 points/line. The central processing apparatus 3 conducting the processing from Step R1 to R5 is an example of the plane-projected data calculating means of the present invention.

At Step R6, the high-density image-positional line data Dh are sampled and interpolated/extrapolated, if necessary, to generate back-projected pixel data D2 for pixels on the lines L0 - L8, as shown in Figure 12.

At Step R7, the high-density image-positional line data Dh are sampled and interpolated/extrapolated to generate back-projected pixel data D2 for pixels in between the lines L0 - L8, as shown in Figure 13. The central processing apparatus 3 conducting the processing from Step R6 to R7 is an example of the back-projected pixel data calculating means of the present invention.

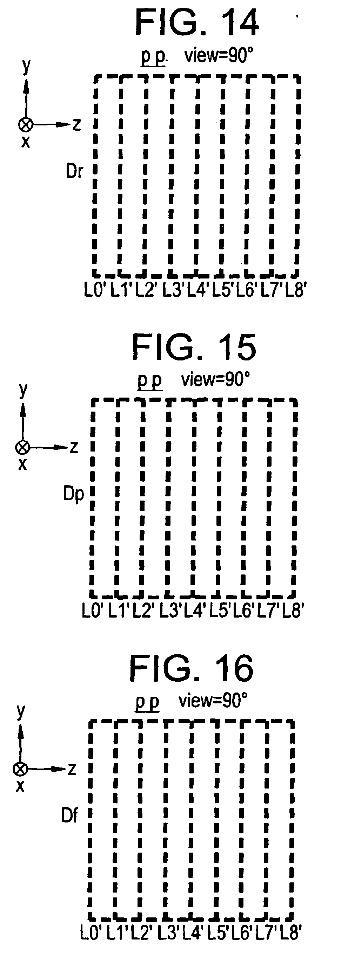

In Figures 8 - 13, -45° ≤ rotation angle < 45° (or a view angle range mainly including the range and also including its vicinity) and 135° ≤ rotation angle < 225° (or a view angle range mainly including the range and also including its vicinity) are assumed, while Figures 14 - 19 are applied for 45° ≤ rotation angle < 135° (or a view angle range mainly including the range and also including its vicinity) and 225° ≤ rotation angle < 315° (or a view angle range mainly including the range and also including its vicinity).

The back-projected pixel data D2 may be determined as the result of weighted addition on back-projected pixel data D2 at a certain rotation angle (view) and back-projected pixel data D2 at an opposite rotation angle (view) with weighting factors *wₐ* and *w_{b} (wₐ* + *w_{b}* = 1) that depend upon the angle formed by a straight line connecting a pixel on the reconstruction field at these views and the X-ray focal spot with respect to the plane of the reconstruction field.

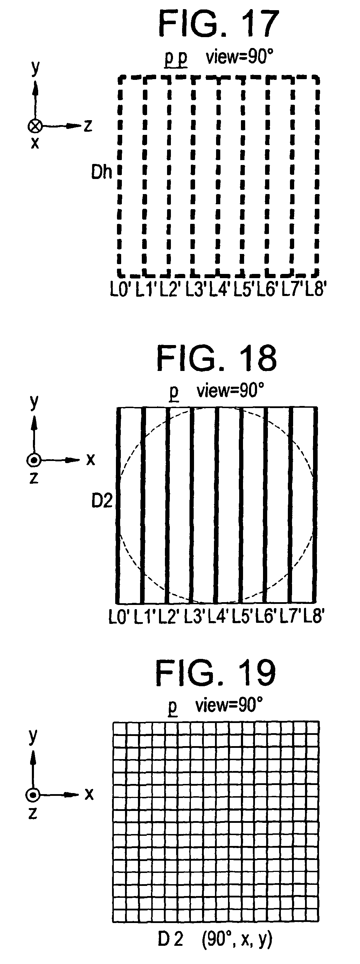

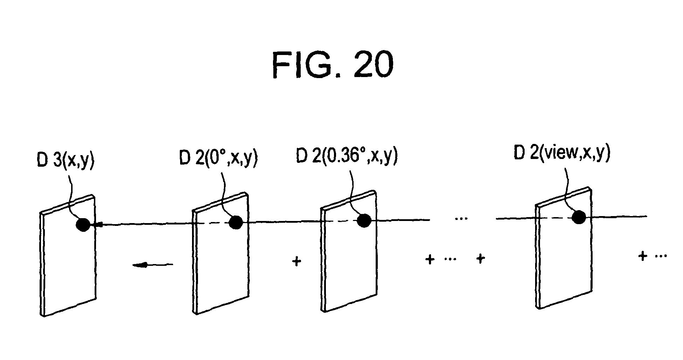

Referring again to Figure 5, at Step R8, the back-projected pixel data D2 shown in Figure 13 or 19 are added on a pixel-by-pixel basis, as shown in Figure 20. At Step R9, Steps R1 - R8 are repeated for all views needed in reconstruction of a CT image (i.e., views for 360° or for "180° + fan angle") to obtain back-projected data D3(x, y). The central processing apparatus 3 conducting the processing from Step R8 to R9 is an example of the back-projected data calculating means of the present invention.

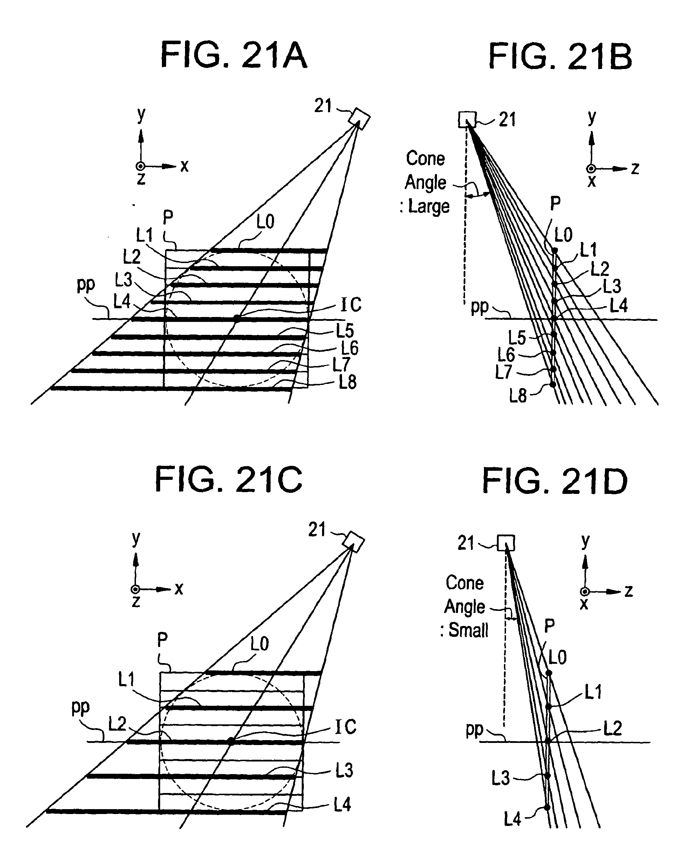

According to the X-ray CT apparatus 100, as shown in Figure 21, when the spacing (indicated by gray bold lines in the drawing) between the planar projection lines on the projection plane pp is to be kept constant for keeping image quality, a larger number of planar projection lines is selected to keep the spacing between the planar projection lines for a larger cone angle formed between the reconstruction plane (X-Y plane) and X-ray beam. For a smaller cone angle, the spacing between the planar projection lines can be kept even if the number of planar projection lines is small.

In other words, for a smaller cone angle formed between the reconstruction plane (X-Y plane) and X-ray beam, the number of planar projection lines may be decreased to reduce the time for reconstruction relative to the case of a larger cone angle. Specifically, for rows near the center in the X-ray multi-row detector, the number of planar projection lines may be decreased to achieve image reconstruction by three-dimensional back-projection at a high speed, and for rows near the edges in the X-ray multi-row detector, the number of planar projection lines may be increased to an extent that does not degrade image quality, thereby reducing the time for image reconstruction processing by three-dimensional back-projection.

Since the reconstruction field around the rows near the edges in the X-ray multi-row detector has some tolerance regarding the signal S/N as compared with the rows near the center, an appropriate filter may be used to attenuate X-rays around the rows near the edges in the X-ray multi-row detector to obtain a uniform S/N. This can also reduce the exposure dose to the subject.

Moreover, different reconstruction kernels may be employed between the rows near the center and those near the edges in the X-ray multi-row detector. In this case, a low-emphasis kernel is used for the rows near the center, and a high-emphasis kernel is used for the rows near the edges. This can also provide a uniform S/N.

Furthermore, the uniform S/N may be obtained by differentiating the number of data elements involved in interpolation between the rows near the center and those near the edges. In this case, the number of data elements involved in interpolation is increased for the rows near the center, and is decreased for the rows near the edges.

The technique for image reconstruction may be a conventionally known three-dimensional image reconstruction technique according to the Feldkamp method. Moreover, three-dimensional image reconstruction techniques proposed in Japanese Patent Application Nos. 2002-066420, 2002-147061, 2002-147231, 2002-235561, 2002-235662, 2002-267833, 2002-322756 and 2002-338947 may be employed.

## Claims

1. A three-dimensional back-projection method (S4) comprising the steps of:
extracting (R2) projection data (Dr) corresponding to a plurality of lines at spacings of a plurality of pixels of a reconstruction field;
generating (R3) projection line data (Dp) by multiplying projection data (Dr) by a cone beam reconstruction weight;
generating (R4) image-positional line data (Df) by filtering the line data (Dp);
generating (R5) high density image-positional line data (Dh) by interpolating the image positional line date (Df) in a line direction;
generating (R6) back-projected data (D2) for pixels on a plurality of lines at spacings of a plurality of pixels of the reconstruction field by sampling the high density image positional line data (Dh);
generating (R7) back-projected data (D2) for pixels in between said plurality of lines at spacings of a plurality of pixels of the reconstruction field by sampling the high density image positional line data (Dh); and
adding (R8) the back-projected data (D2) on a pixel-by-pixel basis for all views needed for image reconstruction (D3).

2. The method of claim 1, wherein generating back-projected data (D2) further comprises interpolating or extrapolating the high density image positional line data (Dh).

3. The three-dimensional back-projection method of claim 1 or claim 2, wherein the number of image positional data lines (Df) is optimized taking image quality of an image to be reconstructed into account.

4. The three-dimensional back-projection method of any one of claims 1 to 3, wherein: representing a direction perpendicular to a plane of rotation of an X-ray tube and an X-ray detector as z-direction, a direction of the center axis of the X-ray beam at a rotation angle of 0° as y-direction, and a direction orthogonal to the z- and y-directions as x-direction, a projection plane is defined as the x-z plane that passes through a center of rotation for -45° s rotation angle < 45° or a rotation angle range mainly including the range and also including its vicinity and 135° ≤ rotation angle < 225° or a rotation angle range mainly including the range and also including its vicinity, and said projection plane is defined as the y-z plane that passes through the center of rotation for 45° s rotation angle < 135° or a rotation angle range mainly including the range and also including its vicinity and 225° ≤ rotation angle < 315° or a rotation angle range mainly including the range and also including its vicinity.

5. A three-dimensional back-projection apparatus (1) comprising means (3) operable to implement the method of any preceding claim.

6. An X-ray CT apparatus (100) comprising:
an X-ray tube (21);
a multi-row detector (24) having a plurality of detector rows;
a scanning device (25) for collecting projection data (D0) while rotating at least one of said X-ray tube and said multi-row detector around a subject to be imaged; and
a three dimensional back-projection apparatus according to claim 5.

7. The X-ray CT apparatus (100) of claim 6, wherein said means (3) is further operable to determine each data element of the back-projected pixel data (D2) by weighted addition on a plurality of data elements of plane-projected data.

8. The X-ray CT apparatus (100) of claims 6 or 7, wherein said means (3) if further operable to determine the back-projected pixel data (D2) as the result of weighted addition on back-projected pixel data (D2) at a certain rotation angle (view) and back-projected pixel data (D2) at an opposite rotation angle (view) with weighting factors wₐ and w_{b} (wₐ + *w_{b}* = 1) that depend upon the angle formed by a straight line connecting a pixel on the reconstruction field at these views and an X-ray focal spot with respect to the plane of the reconstruction field.

## Patentansprüche

1. Dreidimensionales Rückprojektionsverfahren (S4) mit den Schritten:
Gewinnen (R2) von Projektionsdaten (Dr), die mehreren Zeilen in Abständen von mehreren Pixeln eines Rekonstruktionsfeldes entsprechen;
Erzeugen (R3) von Projektionszeilendaten(Dp) durch Multiplizieren von Projektionsdaten (Dr) mit einer Konusstrahl-Rekonstruktionsgewichtung;
Erzeugen (R4) von Bildlage-Zeilendaten (Df) durch Filtern der Zeilendaten (Dp);
Erzeugen (R5) von Bildlage-Zeilendaten (Dh) hoher Dichte durch Interpolieren der Bildlage-Zeilendaten (Df) in einer Zeilenrichtung;
Erzeugen (R6) von rückprojizierten Daten (D2) für Pixel auf mehreren Zeilen in Abständen von mehreren Pixeln des Rekonstruktionsfeldes durch Abtasten der Bildlage-Zeilendaten (Dh) hoher Dichte;
Erzeugen (R7) rückprojizierter Daten (D2) für Pixel zwischen den mehreren Zeilen in Abständen von mehreren Pixeln des Rekonstruktionsfeldes durch Abtasten der Bildlage-Zeilendaten (Dh) hoher Dichte; und
Hinzufügen (R8) der rückprojizierten Daten (D2) auf einer Pixel-für-Pixel-Basis für alle zur Bildrekonstruktion (D3) benötigten Ansichten.

2. Verfahren nach Anspruch 1, wobei das Erzeugen rückprojizierter Daten (D2) ferner den Schritt der Interpolation oder Extrapolation von Bildlage-Zeilendaten (Dh) hoher Dichte aufweist.

3. Dreidimensionales Rückprojektionsverfahren nach Anspruch 1 oder 2, wobei die Anzahl von Bildlage-Zeilendaten (Df) optimiert wird, indem die Bildqualität eines zu rekonstruierenden Bildes berücksichtigt wird.

4. Dreidimensionales Rückprojektionsverfahren nach einem der Ansprüche 1 bis 3, wobei: bei einer Darstellung einer Richtung senkrecht zu einer Rotationsebene einer Röntgenröhre und eines Röntgendetektors als z-Richtung, einer Richtung der Mittenachse des Röntgenstrahls bei einem Rotationswinkel von 0° als y-Richtung, und einer Richtung senkrecht zu der z- und den y-Richtungen als x-Richtung, eine Projektionsebene als die x-z Ebene definiert ist, die durch einen Rotationsmittelpunkt für -45° ≤ Rotationswinkel < 45° oder einen Rotationswinkelbereich, der hauptsächlich diesen Bereich beinhaltet und auch dessen Umgebung beinhaltet, und 135° ≤ Rotationswinkel < 225° oder einen Rotationswinkel, der hauptsächlich diesen Bereich beinhaltet und auch dessen Umgebung beinhaltet, verläuft, und die Projektionsebene als die y-z Ebene definiert ist, die durch den Rotationsmittelpunkt für 45° ≤ Rotationswinkel < 135° oder einen Rotationswinkelbereich, der hauptsächlich diesen Bereich beinhaltet und auch dessen Umgebung beinhaltet, und 225° ≤ Rotationswinkel < 135° oder einen Rotationswinkel, der hauptsächlich diesen Bereich beinhaltet und auch dessen Umgebung beinhaltet, durchläuft.

5. Dreidimensionale Rückprojektionsvorrichtung (1) mit einer Einrichtung (3), die so betrieben werden kann, dass sie das Verfahren nach jedem vorstehenden Anspruch implementiert.

6. Röntgen-CT-Vorrichtung (100), aufweisend:
eine Röntgenröhre (21);
einen Mehrzeilendetektor (24) mit mehreren Detektorzeilen;
eine Scanvorrichtung (25) zum Sammeln von Projektionsdaten (DO), während sich wenigstens eines von der Röntgenröhre und dem mehrzeiligen Detektor um einen abzubildenden Patienten dreht; und
eine dreidimensionale Rückprojektionsvorrichtung nach Anspruch 5.

7. Röntgen-CT-Vorrichtung (100) nach Anspruch 6, wobei die Einrichtung (3) ferner so betrieben werden kann, dass sie jedes Datenelement der rückprojizierten Pixeldaten (D2) durch gewichtete Addition an mehreren Datenelementen von ebenen-projizierten Daten ermittelt.

8. Röntgen-CT-Vorrichtung (100) nach Anspruch 6 oder 7, wobei die Einrichtung (3) ferner so betrieben werden kann, dass sie die rückprojizierten Pixeldaten (D2) als das Ergebnis gewichteter Addition von rückprojizierten Pixeldaten (D2) bei einem bestimmten Rotationswinkel (Ansicht) und rückprojizierter Pixeldaten (D2) bei einem gegenüberliegenden Rotationswinkel (Ansicht) mit Gewichtungsfaktoren *wₐ* und *w_{b}* (*wₐ* + *w_{b}* =1) ermittelt, die von dem Winkel abhängen, der durch eine gerade Linie gebildet wird, die ein Pixel auf dem Rekonstruktionsfeld bei diesen Ansichten und einen Röntgenbrennpunkt in Bezug auf die Ebene des Rekonstruktionsfeldes verbindet.

## Revendications

1. Procédé (S4) de rétroprojection tridimensionnelle comprenant les étapes consistant à :
extraire (R2) des données (Dr) de projection correspondant à une pluralité de lignes à des espacements d'une pluralité de pixels d'un champ de reconstruction ;
générer (R3) des données (Dp) de ligne de projection en multipliant des données (Dr) de ligne de projection par une pondération de reconstruction du faisceau conique ;
générer (R4) des données (Df) de ligne d'image positionnelle en filtrant les données (Dp) de ligne ;
générer (R5) des données (Dh) de ligne d'image positionnelle à haute densité en interpolant les données (Df) de ligne d'image positionnelle dans une direction de ligne ;
générer (R6) des données rétroprojetées (D2) pour des pixels sur une pluralité de lignes à des espacements d'une pluralité de pixels du champ de reconstruction en échantillonnant les données (Dh) de ligne d'image positionnelle à haute densité ;
générer (R7) des données rétroprojetées (D2) pour des pixels intermédiaires entre ladite pluralité de lignes à des espacements d'une pluralité de pixels du champ de reconstruction en échantillonnant les données (Dh) de ligne d'image positionnelle à haute densité ; et
ajouter (R8) les données rétroprojetées (D2) sur une base pixel par pixel pour toutes les vues nécessaires à la reconstruction (D3) de l'image.

2. Procédé selon la revendication 1, dans lequel générer des données rétro-projetées (D2) inclut en plus d'interpoler ou d'extrapoler les données (Dh) de ligne d'image positionnelle à haute densité.

3. Procédé de rétroprojection tridimensionnelle selon la revendication 1 ou selon la revendication 2, dans lequel le nombre de lignes (Df) de données d'image positionnelles est optimisé en tenant compte de la qualité image d'une image à reconstruire.

4. Procédé de rétroprojection tridimensionnelle selon l'une des revendications 1 à 3, **caractérisé en ce que** : représentant une direction perpendiculaire à un plan de rotation d'un tube à rayons X et d'un détecteur de rayons X en tant que direction z, une direction de l'axe central du faisceau de rayons X sous un angle de rotation de 0° en tant que direction y et une direction orthogonale par rapport aux directions z et y en tant que direction x, un plan de projection est défini en tant que le plan x-z qui passe par un centre de rotation pour -45° ≤ angle de rotation < 45° ou une gamme d'angle de rotation incluant principalement la gamme et incluant également sa proximité et 135° ≤ angle de rotation < 225° ou une gamme d'angle de rotation incluant principalement la gamme et incluant également sa proximité, et ledit plan de projection est défini en tant que le plan y-z qui passe par le centre de rotation pour 45° ≤ angle de rotation < 135° ou une gamme d'angle de rotation incluant principalement la gamme et incluant également sa proximité et 225° ≤ angle de rotation < 315° ou une gamme d'angle de rotation incluant principalement la gamme et incluant également sa proximité.

5. Appareil (1) de rétroprojection tridimensionnelle comprenant un moyen exploitable (3) pour exécuter le procédé de toute revendication précédente.

6. Appareil (100) de CT par rayons X comprenant :
un tube (21) à rayons X ;
un détecteur multi-rang (24) ayant une pluralité de rangs détecteurs ;
un dispositif (25) de scannage pour collecter des données (D0) de projection tout en faisant tourner au moins un dudit tube à rayons X ou dudit détecteur multi-rang autour d'un sujet à imager ; et
un appareil de rétroprojection tridimensionnelle selon la revendication 5.

7. Appareil (100) de CT par rayons X selon la revendication 6, dans lequel ledit moyen (3) est de plus exploitable pour déterminer chaque élément de donnée des données pixel rétroprojetées (D2) par ajout pondéré à une pluralité d'éléments de donnée des données rétroprojetées.

8. Appareil (100) de CT par rayons X selon la revendication 6 ou 7, dans lequel ledit moyen (3) est de plus exploitable pour déterminer les données pixel rétroprojetées (D2) en tant que le résultat d'un ajout pondéré à des données pixel rétroprojetées sous un certain angle (vue) de rotation et des données pixel rétroprojetées (D2) sous un angle (vue) de rotation avec des facteurs de pondération wₐ et *w_{b} (wₐ* + *w_{b}* = 1) qui dépendent de l'angle formé par une ligne droite reliant un pixel au champ de reconstruction dans ces vues et d'une tache focale de rayons X par rapport au plan du champ de reconstruction.
